# EUROPEAN PATENT APPLICATION

(11) **EP 2 546 372 A2**
(43) Date of publication of application: **16.01.2013**
(21) Application number: 12168913.7
(22) Date of filing: 21.10.2008
(51) Int. Cl.: C22C 14/00, C23F 1/26

(54) **Metallic component for medical, surgical and implantological use**

(62) Divisional of application: 08425676.7
(71) Applicant: Keystone Dental, Inc., Burlington, MA 01803 (US)
(72) Inventor: Sandrini, Enrico, 25060 Cellatica - BS (IT)
(74) Representative: Romano, Giuseppe

(57) **Abstract**

The present invention relates to a metallic component (2), made of titanium, alloys containing titanium, alloys containing titanium oxides, for medical, surgical and implantological use, characterized in that it comprises, at least on a portion of its outer surface (3), a predefined coloring and titanium oxide in the form of anatase, wherein the amount of anatase is such as to give the metallic component an antibacterial behavior.

## Description

The present invention relates to a surface treatment for metals, particularly for titanium, alloys containing titanium, alloys containing titanium oxides, for medical, surgical and implantological use.

It is known to resort to metallic materials to provide prostheses, bone implants and dental implants.

In particular, the adoption of titanium to provide these elements is particularly widespread: this material is used both in the pure state and in alloys with other metals or in solid compounds (of various kinds) comprising titanium oxides.

Titanium is the chemical element of the periodic table of elements whose symbol is Ti and whose atomic number is 22.

Titanium is a metallic element that is well-known for its resistance to corrosion (almost as much as platinum) and for its high strength/weight ratio. It is lightweight, strong, with a low density (40% than that of steel). In the pure state, it is rather ductile, shiny, with a metallic white color. However, titanium alloys are not easily workable, and the difficulty in working on machine tools is comparable to that of stainless steel, which is notoriously the most problematic to shape by chip-forming.

Titanium has the property of being biocompatible and therefore is physiologically inert. For this reason, many titanium-based alloys are used in the prosthetic components of the hip and knee, and in dental implant prostheses. However, due to the high friction coefficient, it is never used as an articular joint component.

Again due to its biological inertness and mechanical strength, it is used in the sanitary field to manufacture surgical clips for permanent suture and in dentistry to provide dental implants.

In the case of dental implants, in addition to optimizing the shape and dimensions of the implant itself, it is necessary to adopt colors that make the implant as scarcely visible as possible.

It is known to resort to many techniques for surface coloring of the titanium alloy (or pure titanium): in any case, these techniques yield a highly variable result, since the resulting coloring is practically never constant.

In particular, soft colors, such as for example pink, which is adapted to blend in with the mucous membranes of the mouth of the patient on which the implant will be placed, are difficult to obtain.

The adoption of actual layerings of pigment on the surface of the implant, by means of which it would be possible to achieve the desired coloring with excellent repeatability, is to be avoided, since they become interposed between the tissues of the patient and the titanium, isolating it and rendering useless its biocompatibility.

Methods for chemical and electrochemical coloring have been studied since the 1970s: these methods, without exception, do not allow to obtain a uniform and constant coloring of the entire production run, compromising the overall quality of the production run.

Another important advantage of the use of titanium is given by a particular mineral form of its oxide: anatase or octahedrite (the other two are brookite and rutile).

Anatase is always in the form of small, isolated crystals that have developed clearly, and crystallizes in the tetragonal system.

The antibacterial activity of anatase is known. Hence the use of titanium covered by a film of anatase in the biomedical field both for prostheses (for example for osteointegrated dental implants, to contrast the adhesion of bacterial plaque) and to provide aseptic environments (the walls of an operating room, for example).

A further application relates to the anodic oxidation at high potentials with direct production of a film of anatase that incorporate calcium and phosphorus. These films facilitate osteointegration for example of orthopedic prostheses and avoid the formation of fibrous connective tissue at the bone-implant interface, a condition that is necessary in order to have stable and durable implants.

This technique is disclosed for example in "Osteointegration of Titanium and its Alloys by Anodic Spark Deposition and Other Electrochemical Techniques" A Review - R. Chiesa, E. Sandrini et al - Journal of Applied Biomaterials & Biomechanics 2003;1 :p.91-107.

The formation of a stable layer that has an effective antibacterial action on a portion of titanium (or an alloy thereof) is particularly complex and difficult to obtain.

In this case also, it is particularly complex to achieve a constant presence of anatase on the surface of the implants, and this compromises the quality level of these productions.

The methods for synthesizing anatase by chemical and/or electrochemical means that are known in the literature, while ensuring the formation or deposition of anatase in crystalline and nanocrystalline form, do not allow to combine this titanium oxide phase with an aesthetic form that is equivalent to that of gums (pink, indeed).

The methods that instead describe in the literature the coloring of titanium instead do not allow to obtain the pink (gum-like) coloring by electrochemical means with industrial repeatability, and most of all do not allow to associate with this coloring the presence of anatase and therefore obtain the consequent bacteriostatic properties that are particularly suitable for medical and surgical applications.

The aim of the present invention is to solve the above-mentioned drawbacks, by providing a surface treatment for titanium, alloys containing titanium, alloys containing titanium oxides, for medical, surgical and implantological use, adapted to prepare the surface of the metal for coloring with a constant and repeatable tone over large samples and adapted to form oxides with an antibacterial action on the surface of the treated metal.

Within this aim, an object of the invention is to provide a surface treatment for titanium, alloys containing titanium, alloys containing titanium oxides, for medical, surgical and implantological use, that is suitable for producing uniform specimens of products having a pink color and comprising anatase on the outer surface.

Another object of the invention is to provide a surface treatment for titanium, alloys containing titanium, alloys containing titanium oxides, for medical, surgical and implantological use, that is adapted to produce uniform specimens of products having a white color and comprising anatase on the outer surface.

Another object of the invention is to provide a method that can be obtained easily starting from commonly commercially available elements and materials.

Another object of the invention is to provide a method that can be performed with low costs.

This aim and these and other objects, which will become better apparent hereinafter, are achieved by a surface treatment for titanium, alloys containing titanium, alloys containing titanium oxides, for medical, surgical and implantological use, which comprises the steps of:
a) immersing the metallic component in a first acid solution whose total pH value is comprised between 1 and 4, for a time comprised between ten seconds and five minutes at a temperature comprised between 10°C and 50°C;
b) subjecting the metallic component to washing with water whose total pH value is comprised between 5 and 7, for a time comprised between ten seconds and ten minutes at a temperature comprised between 10°C and 80°C;
c) immersing the metallic component in a second acid solution whose total pH value is comprised between 1 and 4, for a time comprised between two minutes and thirty minutes at a temperature comprised between 25°C and 60°C;
d) subjecting the metallic component to washing with water whose total pH value is comprised between 5 and 7, for a time comprised between ten seconds and ten minutes at a temperature comprised between 10°C and 80°C;
e) immersing the metallic component in a third acid solution whose total pH value is comprised between 1 and 4, for a time comprised between two seconds and two minutes at a temperature comprised between 10°C and 80°C;
f) subjecting the metallic component to washing with water whose total pH value is comprised between 5 and 7, for a time comprised between ten seconds and ten minutes at a temperature comprised between 10°C and 80°C;
g) immersing the metallic component in a fourth acid solution whose total pH value is comprised between 1 and 6, at a temperature comprised between 3°C and 20°C, applying a voltage ramp to the fourth solution.

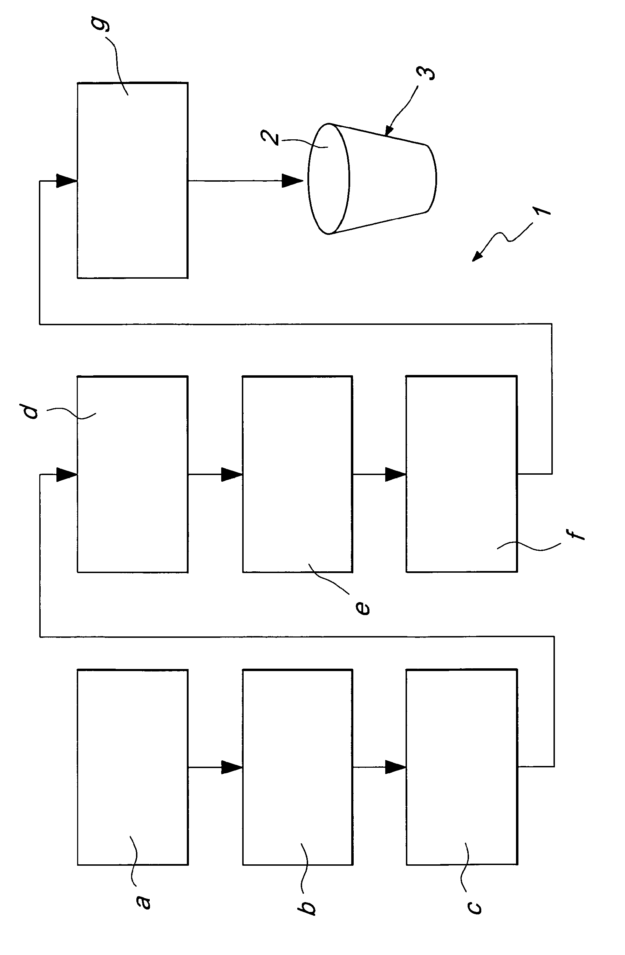

Further characteristics and advantages of the invention will become better apparent from the following detailed description of a preferred but not exclusive embodiment of the surface treatment for titanium, alloys containing titanium, alloys containing titanium oxides, for medical, surgical and implantological use, according to the invention, illustrated by way of non-limiting example in the accompanying drawing, in which the sole figure is a schematic view of a flowchart of the steps of the surface treatment for titanium, alloys containing titanium, alloys containing titanium oxides, for medical, surgical and implantological use, according to the invention.

With reference to the figure, the reference numeral 1 generally designates a surface treatment for titanium, alloys containing titanium, alloys containing titanium oxides, for medical, surgical and implantological use.

### The treatment 1 consists of the following steps.

A step a), which provides for the immersion of a metallic component 2 in a first acid solution whose total pH value is comprised between 1 and 4, for a time comprised between ten seconds and five minutes at a temperature comprised between 10°C and 50°C.

In particular, the preferred duration for step a) is comprised by way of indication in the interval between 45 seconds and 1 minute and 15 seconds, the preferred pH value lies in the neighborhood of 2 and the preferred temperature for this step a) is, by way of indication, 20-30°C.

This first step a) is a step for chemical pickling (cleaning of a metallic surface by using degreasing and/or acid agents) and removal of loose titanium oxides that are present on the surface of the metallic component 2.

A second step b) provides for subjecting the metallic component 2 to washing with water whose total pH value is comprised between 5 and 7, for a time comprised between ten seconds and ten minutes at a temperature comprised between 10°C and 80°C.

100% deionized water by volume, with a pH value of approximately 6, is preferably adopted; washing will last preferably approximately one minute and will occur substantially at ambient temperature.

Step b) is a simple step for rinsing that is adapted to remove any residue of step a).

A third step c) consists in immersing the metallic component 2 in a second acid solution whose total pH value is comprised between 1 and 4, for a time comprised between two minutes and thirty minutes at a temperature comprised between 25°C and 60°C.

In particular, the preferred duration for step c) is comprised by way of indication in the interval between 1 minute and 30 seconds and 2 minutes and 30 seconds, the preferred pH value lies in the neighborhood of 2 and the preferred temperature for this step c) is, by way of indication, 35-45°C.

Step c) is an actual chemical preparation process, by means of which it is possible to obtain a chemical-physical modification of the surface of the metallic component 2, which will make it adapted to perform the aim of the present invention.

At this point, it is necessary to specify that the second acid solution comprises a combination of at least one reducing acid with at least one oxidizing acid, for example of the type of the combination of oxalic acid with sodium fluoride.

More specifically, the second acid solution can comprise oxalic acid with a concentration comprised between 0.01 M and 8 M, which corresponds to the saturated solution, and sodium fluoride with a concentration comprised between 2 mM and 120 mM. Within these ranges, it is noted that a preferred solution for providing the second acid solution has a concentration of, by way of indication, 1 M of oxalic acid and a concentration of, by way of indication, 10 mM of sodium fluoride: in any case, the possibility to adopt different concentrations, with values that are more or less close to the ones indicated as preferred, is not excluded.

During a subsequent step d) it is necessary to subject the metallic component 2 to washing with water having a total pH value comprised between 5 and 7, for a time comprised between ten seconds and ten minutes, at a temperature comprised between 10°C and 80°C.

In particular, for this rinse 100% deionized water by volume with a pH value of approximately 6 is used; washing preferably lasts approximately one minute and occurs substantially at ambient temperature.

Step d) is a simple rinsing step that is adapted to remove any residue of step c).

A step e) provides for the immersion of the metallic component 2 in a third acid solution whose total pH value is comprised between 1 and 4, for a time comprised between two seconds and two minutes, at a temperature comprised between 10°C and 80°C.

It should be noted that the first acid solution and the third acid solution comprise a combination of at least one reducing acid with at least one oxidizing acid, for example of the type of the combination of hydrofluoric acid with nitric acid.

More specifically, the first acid solution and the third acid solution comprise hydrofluoric acid in a percentage by volume comprised between 1% and 8% and nitric acid in a percentage by volume comprised between 15% and 45%. Attention is called to the preferred adoption, for the first and third acid solutions, of hydrofluoric acid in a percentage by volume of, by way of indication, 3% and of nitric acid in a percentage by volume of, by way of indication, 24% (the possibility to adopt other values according to different types of treatment is in any case not excluded).

Step e) also (like step a)) is a step for chemical pickling (cleaning of a metallic surface by using degreasing and/or acid agents) and for removal of loose titanium oxides that are present on the surface of the metallic component 2.

A subsequent step f) entails subjecting the metallic component 2 to a further wash with water whose total pH value is comprised between 5 and 7, for a time comprised between ten seconds and ten minutes at a temperature comprised between 10°C and 80°C.

In this case also, 100% deionized water by volume with a pH value of approximately 6 is preferably used; washing lasts preferably approximately one minute and occurs substantially at ambient temperature.

Step f) is a simple rinsing step that is adapted to remove any residue of step e).

According to a final step g), it is necessary to immerse the metallic component 2 in a fourth acid solution whose total pH value is comprised between 1 and 6, at a temperature comprised between 3°C and 20°C, applying a voltage ramp to the fourth solution.

The fourth acid solution comprises an inorganic acid, with the exception of hydrofluoric and hydrochloric acid, for example of the type of sulphuric acid.

In particular, the fourth acid solution comprises sulphuric acid in a percentage by volume comprised between 0.5% and 50%.

The voltage ramp (typical of step g)) applied to the fourth solution has a rise rate comprised between 1 V/s and 20 V/s, starting from a preset initial voltage value, until it reaches a final voltage value comprised between 40 and 90 (although it is noted that within the subinterval between 58 and 65 V it has been found that it is possible to obtain optimum results). The initial voltage value can be equal to zero or equal to any value that depends on the final result to be obtained. The voltage can be continuous or variable (for example AC, sinusoidal, square-wave, et cetera), and the final value to be reached is preferably equal to approximately 62 V.

The rise rate of the voltage, the parameter that determines the slope of the ramp, is preferably in the neighborhood of approximately 5 V/s: i.e., the voltage increases by 5 V every second, reaching the preferred value of approximately 62 V (assuming to start from a potential of zero) in approximately 12-13 seconds.

It is important to note that the metallic component 2 to be treated undergoes a preventive degreasing and a complete removal of machining residues, both for metallic flash and for machining oil. These preventive activities prepare the metallic component 2 for the treatment 1, optimizing its effectiveness.

The metallic component 2 can be constituted by titanium (particularly pure titanium in the alpha phase), alloys containing titanium (for example titanium-based alloys in the alpha phase and in the beta phase, beta-phase titanium alloys, and alpha-phase titanium alloys), alloys containing titanium oxides.

Mainly, in order to provide metallic components 2 for medical, surgical and implantological use, pure grade 4 alpha-phase titanium is used.

The metallic component 2 that has undergone the treatment 1 according to the invention comprises, at least on a portion of its outer surface 3, a predefined coloring and titanium oxide in the form of anatase. In particular, the amount of anatase is such as to give the metallic component 2 an antibacterial behavior, hindering, in the case of the provision of dental implants, the formation of plaque on the outer surface 3.

It is convenient to note that the predefined coloring is a tone of pink that is similar to the color of gums.

Advantageously, by adopting the treatment 1 according to the invention, the resulting coloring has a constant tone and the metallic components 2 of a same color can be provided with absolute repeatability both as regards the tone of color and as regards the anatase content on the outer surface 3.

The described treatment 1, by widening the range of electrochemical parameters within which it is possible to achieve the coupling of the aesthetic properties of the "gum pink" color, constituted by titanium oxide in anatase phase, repeatably and in a manner that allows industrialization, thus allows to apply this technology to articles designed for medical and surgical applications, combining the functional properties of anatase and the aesthetic properties of the pink coloring repeatably and in a manner that allows industrialization, something which was not plausible with currently known methods.

In the exemplary embodiments shown, individual characteristics, given in relation to specific examples, may actually be interchanged with other different characteristics that exist in other exemplary embodiments.

In practice, the materials used, as well as the dimensions, may be any according to requirements and to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A metallic component (2), made of titanium, alloys containing titanium, alloys containing titanium oxides, for medical, surgical and implantological use, **characterized in that** it comprises, at least on a portion of its outer surface (3), a predefined coloring and titanium oxide in the form of anatase, wherein the amount of anatase is such as to give the metallic component an antibacterial behavior.

2. The metallic component according to claim 1, **characterized in that** said predefined coloring is a tone of pink similar to the color of gums.

3. The metallic component according to claim 1 or 2, **characterized in that** said coloring is due to the color of the anatase distributed on the outer surface (3) of the metallic component (2).

4. The metallic component according to one of the preceding claims, wherein said titanium is pure titanium in the alpha phase.

5. The metallic component according to claim 4, wherein said pure titanium in the alpha phase is pure grade 4 alpha-phase titanium.

6. The metallic component according to one of the preceding claims, wherein said alloys containing titanium are titanium-based alloys in the alpha phase and in the beta phase, beta-phase titanium alloys, and alpha-phase titanium alloys.
